# EUROPÄISCHE PATENTSCHRIFT

(11) **EP 0 668 264 B1**
(45) Veröffentlichungstag und Bekanntmachung des Hinweises auf die Patenterteilung: **22.09.1999**
(21) Anmeldenummer: 95101402.6
(22) Anmeldetag: 02.02.1995
(51) Int. Cl.: C07C 323/52, C07C 323/53, C03C 17/10

(54) **Schwefelorganische Goldverbindungen, Verfahren zu ihrer Herstellung und Verwendung derselben**
Sulfur containing organic gold compounds, process for their preparation and their use
Composés d'or organiques soufrés, procédé pour leur préparation et leur utilisation

(30) Priorität: 21.02.1994 DE 4405424
(43) Veröffentlichungstag der Anmeldung: 23.08.1995
(73) Patentinhaber: CERDEC AKTIENGESELLSCHAFT KERAMISCHE FARBEN, D-60327 Frankfurt (DE)
(72) Erfinder: Schulz, Andreas, Dr., D-63263 Neu-Isenburg (DE); Höfler, Marco, D-63579 Freigericht (DE)

(56) Entgegenhaltungen:
- EP-A- 0 514 073
- DE-C- 949 055
- GB-A- 662 015
- GB-A- 716 647

## Beschreibung

Die Erfindung richtet sich auf neuartige schwefelorganische Goldverbindungen sowie ein Verfahren zu ihrer Herstellung. Ein weiterer Gegenstand richtet sich auf die Verwendung der neuen Verbindungen zur Herstellung von Mitteln zur Erzeugung eines Golddekors auf einbrennfähigen Unterlagen.

Zur Herstellung eines Golddekors, worunter auch Leiterbahnen integrierter Schaltkreise zählen, auf einem einbrennfähigen Substrat, wie insbesondere Glas, Porzellan und Keramik finden seit langem Goldpräparate, darunter sogenannte Glanzgoldpräparate und Poliergoldpräparate Anwendung. Solche Präparate enthalten im allgemeinen eine oder mehrere schwefelorganische Goldverbindungen, ein oder mehrere organische polymere Bindemittel und ein Lösungsmittelsystem. Zusätzlich enthalten die Präparate zur Einstellung gewünschter optischer und Gebrauchseigenschaften des herzustellenden Dekors andere lösliche und/oder unlösliche Edelmetallverbindungen und/oder Gold und ein oder mehrere Flußmittel, ferner zur Einstellung der Verarbeitungseigenschaften derartiger Präparate weitere Hilfsstoffe. Die Edelmetallpräparate werden mittels üblicher direkter und indirekter Druckverfahren, Sprühen oder Pinseln oder unter Verwendung der Abziehbildtechnik auf die zu beschichtende Oberfläche aufgetragen. Nach dem Abdunsten des Lösungsmittels schließt sich ein Brennvorgang bei einer auf das Substrat und das Goldpräparat abgestimmten Temperatur an - meistens liegt die maximale Brenntemperatur zwischen 400 und 900 °C, jedoch sind in Sonderfällen auch höhere Temperaturen möglich. Durch den Brennvorgang wird ein Edelmetallfilm ausgebildet und auf der Oberfläche des Substrats fixiert.

Bei den schwefelorganischen Goldverbindungen für die genannten Dekorationspräparate handelte es sich lange Zeit fast ausschließlich um sogenannte Goldsulforesinate, welche aus einem Gold(III)-salz und einem geschwefelten, insbesondere natürlich vorkommenden Terpen, gewonnen wurden. In diesen sowie in synthetischen Gold-thiolaten der allgemeinen Formel Au-S-R, worin R für eine Alkyl-, Cycloalkyl-, Aryl- oder Aralkylgruppe oder einen bicyclischen Kohlenwasserstoffrest steht, liegt das Gold in der einwertigen Form vor. Die vorgenannten Goldverbindungen erfordern bei der Verwendung in Goldpräparaten die Verwendung eines rein organischen Lösungsmittelsystems.

Aus arbeitshygienischen, Sicherheits- und Umweltgründen besteht ein wachsendes Interesse an Edelmetallpräparaten zum Dekorieren einbrennfähiger Substrate, in deren Lösungsmittelsystem zumindest ein Teil des organischen Lösungsmittels durch Wasser ersetzt ist. So ist aus der DE-OS 32 17 049 ein Anstrichmittel zum Auftragen einer Überglasurdekoration auf Porzellan bekannt, das 15 bis 40 Gew.-% Polyvinylpyrrolidon oder ein Gemisch aus Polyvinylpyrrolidon und wäßrigem Polyethylenoxid, 45 bis 85 Gew.-% Ethylenglykol und/oder Propylenglykol und eventuell Wasser enthält. Als färbende Stoffe werden in diesem Dokument Oxide, Gold und organische Goldverbindungen genannt, ohne jedoch irgendeine Struktur der Goldverbindungen anzugeben.

Aus der EP-A 0 514 073 sind homogene Zusammensetzungen, vorzugsweise Lösungen, bekannt, welche beim Einbrennen einen glänzenden metallischen Edelmetallfilm ausbilden. Die Zusammensetzungen enthalten 3 bis 22 Gew.-% eines Edelmetall-thiolats, ein polymeres Harz und als Lösungsmittelsystem ein Gemisch aus Wasser und einem organischen Lösungsmittel (Cosolvens), bei welchem es sich vorzugsweise um wassermischbare Alkohole, Ether oder Ester handelt. Sowohl das Edelmetall-thiolat als auch das Bindemittel sollen in dem Wasser/Cosolvens-Gemisch löslich sein. Bei den bevorzugt eingesetzten Gold(I)-thiolaten handelt es sich um solche der allgemeinen Formel Au-S-R-H oder Au-S-R-X, wobei X für eine Nitrogruppe oder -COOH, -SO₂OH, -OH, -CONH₂, -NH₂ oder -O-P(O)(OH)₂, wobei die H-Atome gegebenenfalls substituiert sein können, oder Salze davon und R für einen divalenten organischen Rest stehen. Aus dem genannten Dokument geht anhand zahlreicher Beispiele und Vergleichsbeispiele deutlich hervor, daß nur ganz spezielle Gold(I)-thiolate in entsprechenden Wasser und Cosolvens enthaltenden Goldpräparaten zu Dekoren mit gutem Glanz und guter Haftfestigkeit auf der dekorierten Unterlage führen; mit anderen unter die genannten Formeln fallenden Gold(I)-thiolaten werden demgegenüber nur matte und/oder schlecht haftende Dekore erhalten.

Aufgabe der vorliegenden Erfindung ist demgemäß, eine neue Gruppe von schwefelhaltigen Goldverbindungen aufzuzeigen, welche sich in Wasser enthaltenden Präparaten zur Herstellung von Golddekoren, insbesondere hochglänzenden Golddekoren, eignen.

Es wurde gefunden, daß Monogold(I)-dimercaptobernsteinsäure und ähnlich strukturierte schwefelorganische Goldverbindungen nach Auflösen in Wasser in Gegenwart einer Base, vorzugsweise eines Amins, und anschließendes Ansäuern nicht in Form der ursprünglichen Verbindung ausgefällt werden, sondern überraschenderweise in Form völlig neuer schwefelorganischer Goldverbindungen. Die wie vorstehend skizziert erhältlichen neuen schwefelorganischen Goldverbindungen sind in Gegenwart einer Base wasserlöslich; aus derartigen Lösungen sind unmittelbar wasserlösliche erfindungsgemäße schwefelorganische Goldverbindungen erhältlich.

Die erfindungsgemäßen schwefelorganischen Goldverbindungen sind gekennzeichnet durch einen Goldgehalt im Bereich von 60 bis 90 Gew.-% und ein Atomverhältnis von Gold zu Schwefel von größer 0,7 bis 4. Die erfindungsgemäßen schwefelorganischen Goldverbindungen sind durch das nachfolgend beschriebene Verfahren erhältlich. Vorzugsweise weisen die Goldverbindungen einen Goldgehalt zwischen 68 und 85 Gew.-%, insbesondere 73 bis 80 Gew.-%, und ein Atomverhältnis von Gold zu Schwefel von größer 1 bis 3, insbesondere 1,2 bis 2, auf und sind in Wasser, gegebenenfalls nach Zugabe einer Base, gut löslich.

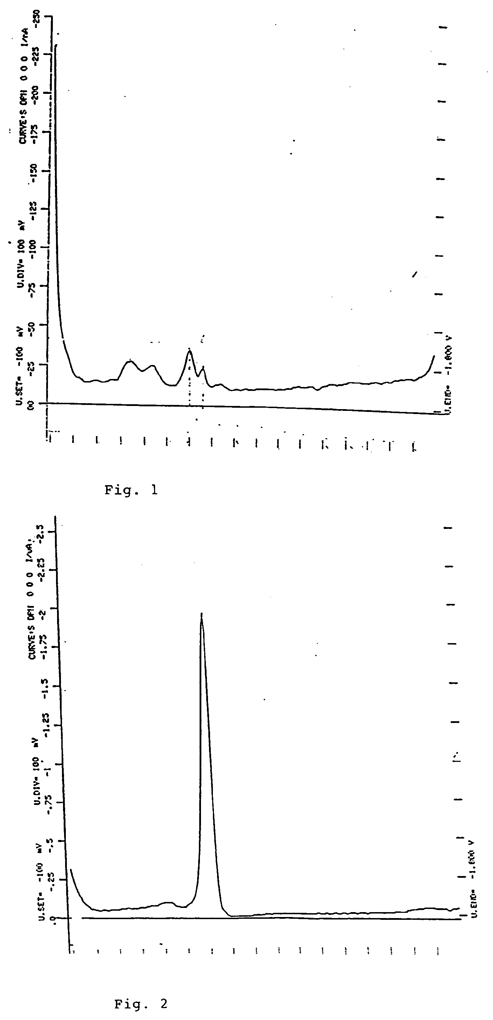

Die neuen schwefelorganischen Goldverbindungen weisen Eigenschaften auf, die sie von den Ausgangsverbindungen zu ihrer Herstellung prinzipiell unterscheiden. So lassen sich die neuen Verbindungen elektrochemisch bis -1,8 Volt nicht mehr reduzieren. Dies ist überraschend, weil Gold(I)-thiolate wie die in der EP-A 0 514 073 genannten Gold(I)-mercaptopropionylglycin und Gold(I)-mercaptobernsteinsäure bei der polarographischen Bestimmung in 0,05 molarer Natronlauge ein Halbwellen-Reduktionpotential von -550 mV gegen Silber/Silberchlorid aufweisen; auch die zur Herstellung der neuen Goldverbindungen besonders geeignete Monogold(I)-dimercaptobernsteinsäure läßt sich problemlos elektrochemisch reduzieren - Halbwellenpotential in 0,5 molarer Natronlauge -700 mV gegen Ag/AgCl. Die elektrochemische Nicht-Reduzierbarkeit der neuen Goldverbindungen im Vergleich zu Gold(I)-thiolaten ist ein Indiz dafür, daß es sich bei den neuen Verbindungen nicht um Gold(I)-thiolate handelt, sondern um Au-Verbindungen mit einer Wertigkeit des Goldes von kleiner +1. Figur 1 zeigt das Voltammogramm der erfindungsgemäßen Goldverbindung gemäß Beispiel 3, Figur 2 zum Vergleich das Voltammogramm der Ausgangsverbindung gemäß Beispiel 1 - zu beachten ist, daß die Ordinate in Fig. 1 nA, diejenige in Fig. 2 aber µA angibt.

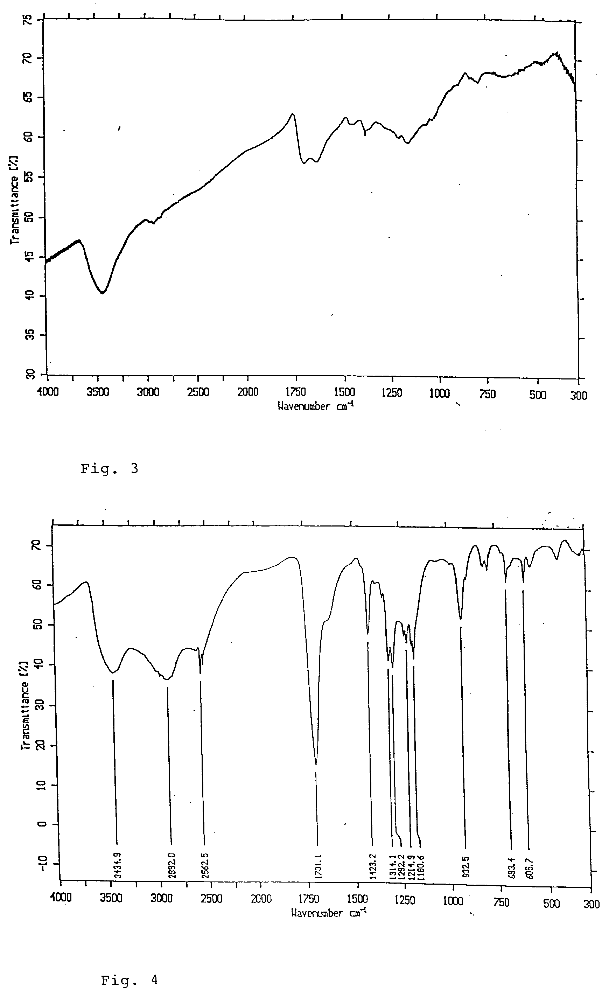

Mittels Röntgenphotoelektronenspektroskopie/XPS/ESCA wurde bei hoher Spektrometerauflösung und Verwendung einer gesputterten Reinstgoldprobe als Kalibrierungsnormal das Maximum des Au4f_{7/2}-Peaks von Monogold(I)-dimercaptobernsteinsäure und der daraus hergestellten erfindungsgemäßen schwefelorganischen Goldverbindung (Beispiel 3) im Vergleich zu elementarem Gold bestimmt: Das Maximum des genannten Peaks der erfindungsgemäßen Verbindung liegt mit 84,2 eV zwischen den Werten für metallisches Gold (84,0 eV) und dem als Ausgangsmaterial eingesetzten Gold(I)-thiolat (Beispiel 1)(84,46 eV). Die kleine, jedoch signifikante Signalverschiebung des Au4f_{7/2}-Peaks zu gegenüber dem metallischen Gold höheren Bindungsenergien wird als Hinweis auf das Vorliegen eines Gold-Clusters mit einer Wertigkeit des Goldes von kleiner +1 und größer 0 gewertet. Als weitere Indizien hierfür können der sehr hohe Goldgehalt sowie das hohe Atomverhältnis Au zu S angesehen werden. Auch bezüglich der IR-Spektren unterscheiden sich die Ausgangsverbindungen grundlegend von denen der erfindungsgemäßen Verbindungen - Figur 3 zeigt das IR-Spektrum des Produkts von Beispiel 3 und Figur 4 das IR-Spektrum des Ausgangsproduktes (Beispiel 1).

Die erfindungsgemäßen Goldverbindungen lassen sich durch ein Verfahren herstellen, das die folgenden Stufen umfaßt:
(i) Auflösen einer Monogold(I)-dimercaptocarbonsäureverbindung der allgemeinen Formel (A) 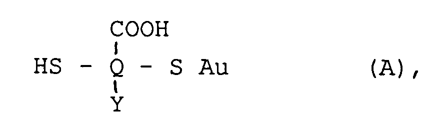 worin Q einen aliphatischen C₂- bis C₄-Alkantetraylrest oder einen 5- oder 6-gliedrigen, gegebenenfalls ein Sauerstoff- oder Imin-Ringglied enthaltenden cycloaliphatischen Tetraylrest und Y Wasserstoff oder COOH bedeuten und die S-Atome an benachbarten C-Atomen stehen, oder ein Salz der Verbindung (A) in Wasser in Gegenwart einer Base, vorzugsweise eines Amins,
(ii) Zugabe einer Mineralsäure zur Lösung der Stufe (i) bis zu einem pH-Wert von gleich oder unter 2 und
(iii) Isolierung des in Stufe (ii) ausgefallenen Produkts.

Die Gruppe Y steht bevorzugt für eine Carboxylgruppe. Damit sind besonders bevorzugte Monogold(I)-dimercaptocarbonsäure-Ausgangsstoffe der Formel (A) Monogold(I)-dimercaptobernsteinsäure, Monogold(I)-2,3-dimercaptoglutarsäure und Monogold(I)-2,3- oder -3,4-dimercaptoadipinsäure; unter den cyclischen Ausgangsverbindungen der Formel (A) werden beispielhaft Monogold(I)-3,4-dimercapto-2,5-dicarbonsäure und Monogold(I)-2,3-dimercapto-cyclohexan-1,4-dicarbonsäure genannt. Die Herstellung der als Ausgangsverbindung dienenden Monogold(I)-dithiolate der Formel (A) erfolgt in prinzipiell analoger Weise, wie dies aus der Herstellung vorbekannter Edelmetall-thiolate der Fachwelt bekannt ist: Aus Tetrachlorogoldsäure wird nach Zugabe der doppelt äquivalenten Menge eines Thioethers der Formel RSR' ein Gold(I)-komplex der Formel AuCl(RSR') gebildet; ein besonders geeigneter Thioether für diese Umsetzung ist Methionin gemäß EP-B 0 491 147. Der genannte Komplex wird sodann in Gegenwart eines Lösungsmittels mit einer im wesentlichen äquivalenten Molmenge des der Verbindung (A) zugrundeliegenden Dithiols (Dimercaptoverbindung) umgesetzt, wobei die Monogoldverbindung gemäß Formel (A) gebildet wird. Die Herstellung der der Verbindung (A) zugrundeliegenden Dithiolverbindung erfolgt unter Heranziehung üblicher Verfahrensstufen: Beispielsweise lassen sich Dimercaptoalkandicarbonsäuren durch Umsetzung der entsprechenden Alkindicarbonsäuren mit Thioessigsäure gewinnen; eine weitere Zugangsmöglichkeit für aliphatische und cyclische Dimercaptoverbindungen, wie sie den Goldverbindungen der Formel (A) zugrundeliegen, besteht darin, die entsprechende Dihalogenverbindung (Y-Q(Hal)₂-COOH) mit einem Alkalisulfid umzusetzen.

Im erfindungsgemäßen Verfahren kann in der Stufe (i) eine beliebige Base, wie etwa Natronlauge oder ein Amin verwendet werden. Bevorzugt werden Amine, worunter primäre, sekundäre und tertiäre Amine mit aliphatischen, cycloaliphatischen und/oder aromatischen Resten und zusätzlich N-heterocyclische Basen, wie insbesondere Pyridin, verstanden werden. Bevorzugt werden tertiäre Amine mit gleichen oder verschiedenen niederen (C₁- bis C₄-)alkylresten; besonders bevorzugt werden Triethylamin, Tri-n-propylamin und Tri-iso-propylamin. In der Stufe (i) wird die Goldverbindung in Wasser eingetragen und eine Base, bevorzugt ein Amin in einer solchen Menge zugesetzt, daß sich die Goldverbindung auflöst. Sofern unmittelbar ein wasserlösliches Salz der Monogold(I)-dimercaptocarbonsäureverbindung der allgemeinen Formel (A) in der Stufe (i) eingesetzt wird, erübrigt sich die Zugabe einer Base, oder die Menge kann gegebenenfalls reduziert werden. Unter Verwendung der Monogoldverbindung der allgemeinen Formel (A) wird durch die Zugabe der Base zweckmäßigerweise ein pH-Wert von mindestens 8, vorzugsweise ein pH-Wert zwischen 9 und 14, eingestellt. Vorzugsweise wird die Monogoldverbindung der allgemeinen Formel (A) in einer solchen Menge eingesetzt, daß in der Lösung ein Goldgehalt zwischen 2 und 20 Gew.-% resultiert.

In der Stufe (ii) wird eine beliebige Mineralsäure, vorzugsweise Salzsäure, zugesetzt, bis ein pH-Wert von 2 oder weniger als 2 erreicht ist. Während oder nach der Zugabe der Mineralsäure fällt ein braunes bis schwarzes Produkt aus, das in bekannter Weise in der Stufe (iii) von der Lösung abgetrennt wird.

Das in der Stufe (iii) isolierte Produkt weist bereits alle Eigenschaften der erfindungsgemäßen schwefelorganischen Goldverbindungen auf. Eine weitere Steigerung des Goldgehalts und Absenkung des Schwefelgehalts läßt sich dadurch erzielen, daß die Stufen (i) bis (iii) ein- oder mehrfach, vorzugsweise ein- oder zweifach, wiederholt werden, wobei als Ausgangsprodukt für die Stufe (i) der zweiten und folgenden Sequenzen jeweils das in der Stufe (iii) der vorhergehenden Sequenz isolierte Produkt eingesetzt wird. Die Reaktionsstufen (i) bis (iii) können bei Raumtemperatur oder erhöhter Temperatur, vorzugsweise bei 30 bis 80 °C, durchgeführt werden.

Die nach dem beschriebenen Verfahren erhältlichen schwefelorganischen Goldverbindungen lösen sich in Wasser in Gegenwart einer Base. Durch Abdampfen des Wassers aus einer so erhaltenen Lösung unter vermindertem Druck werden unmittelbar wasserlösliche schwefelorganische Goldverbindungen mit den für die neue Stoffklasse charakteristischen Eigenschaften erhalten.

Es wurde gefunden, daß sich die erfindungsgemäßen schwefelorganischen Goldverbindungen hervorragend zur Herstellung eines Golddekors auf einbrennfähigen Unterlagen, wie insbesondere Glas, Porzellan und Keramik, eignen. Zweckmäßigerweise werden zur Erzeugung eines Golddekors die schwefelorganischen Goldverbindungen nicht per se, sondern in Form von diese enthaltenden Mitteln, insbesondere in Form sogenannter Glanzgold- und Poliergoldpräparate sowie in Form von Abziehbildern, deren Dekorschicht die schwefelorganischen Goldverbindungen und organische polymere Bindemittel enthält, verwendet.

Die genannten Mittel, darunter sogenannte Glanzgold- und Poliergoldpräparate, zur Erzeugung eines Golddekors auf einer einbrennfähigen Unterlage oder zur Herstellung der Dekorschicht eines Abziehbilds enthalten mindestens eine erfindungsgemäße schwefelorganische Goldverbindung, ein oder mehrere polymere organische Bindemittel sowie ein Lösungsmittelsystem, das die Polymeren und die Goldverbindung löst oder anderweitig eine homogene Verteilung gewährleistet. Soweit erforderlich, enthalten derartige Präparate zusätzlich andere Edelmetallverbindungen in Form einfacher Salze, Komplexe, Resinate oder Thiolate von Silber, Platin und/oder Palladium, um Farbnuancen des gewünschten Dekors einzustellen. Poliergoldpräparate enthalten ferner noch Goldpulver und/oder unlösliche Goldverbindungen. Zusätzlich enthalten die Präparate im allgemeinen Flußmittel in Form von Verbindungen, beispielsweise Resinaten, Salzen, Oxiden oder Koordinationsverbindungen, eines oder mehrerer der Elemente Bor, Silicium, Vanadium, Chrom, Indium, Zinn, Antimon, Wismut oder Rhodium und andere Hilfsstoffe, um die gewünschten Verarbeitungseigenschaften, etwa eine siebdruckfähige Viskosität und/oder eine hohe Trocknungsgeschwindigkeit des Präparats, zu erzielen und ganz bestimmte optische und Gebrauchseigenschaften, wie gute Haftung des eingebrannten Dekors auf dem Untergrund, einzustellen.

Die erfindungsgemäßen schwefelorganischen Goldverbindungen lassen sich in Präparaten verwenden, deren Lösungsmittelsystem rein organisch, organisch-wäßrig oder rein wäßrig ist. Der Fachmann wird die in den Präparaten zu verwendenden Bindemittel und Lösungsmittel aufeinander abstimmen, um eine möglichst homogene Zusammensetzung, vorzugsweise eine Lösung zu erhalten.

Wäßrig-organische oder rein wäßrige Präparate mit einer erfindungsgemäßen schwefelorganischen Goldverbindung enthalten solche polymeren Bindemittel, welche in Wasser löslich sind oder darin eine klare Dispersion bilden. Zweckmäßige Bindemittel sind Polyacrylsäure, Polymethacrylsäure, Polyvinylpyrrolidon, Celluloseether, insbesondere Carboxyalkyl- und Hydroxyalkyl-Cellulose, Polyalkylenglykol, Polyvinylacetat, Polyvinylalkohol, Polyamine, Alkydharze und Polyurethanharze. Die Bindemittel können in Form von Homopolymeren oder Copolymeren oder Blockpolymeren, jeweils einzeln oder in Form von Gemischen zur Anwendung gelangen. Besonders bevorzugt für rein wäßrige Präparate oder solche mit hohem Wassergehalt werden Polyvinylpyrrolidon-homo- oder -copolymere, Polymethacrylsäure-homo- oder -copolymere sowie Harze auf der Basis von Celluloseethern.

Präparate mit einem wäßrig-organischen Bindemittelsystem enthalten als organisches Lösungsmittel ein oder mehrere wasserlösliche organische Lösungsmittel, insbesondere wasserlösliche Alkohole, Ether und Ester. Besonders bevorzugt finden Glykole mit 2 bis 4 C-Atomen sowie Oligo- und Poly(C₂- bis C₄-)glykole oder Mono(C₁- bis C₄-)alkylether der genannten Glykole oder Oligoglykole Verwendung, ferner Hydroxycarbonsäuren oder niedere Alkylester derselben, wie insbesondere C₁- bis C₃-Alkyllactat.

Wie bereits angesprochen, ist es unter Verwendung der erfindungsgemäßen schwefelorganischen Goldverbindungen überraschenderweise auch möglich, im wesentlichen rein wäßrige Präparate (Gehalt an organischen Lösungsmitteln unter 1 Gew.-%) zu erzeugen, indem die erfindungsgemäße Goldverbindung, sofern nötig nach Zugabe eines Amins, in Wasser gelöst und diese Lösung in an sich bekannter Weise mit einer wasserlösliche organische Bindemittel und übrige Hilfsstoffe enthaltenden wäßrigen Lösung oder Emulsion zusammengebracht wird. Um besonders hochglänzende Golddekore zu erzeugen, enthalten derartige wäßrige Präparate zweckmäßigerweise zusätzlich ein anionisches, kationisches, zwitterionisches oder nichtionisches Tensid in wirksamer Menge, meist 0,01 bis 2,0 Gew.-%. Sehr wirksame Tenside sind polyethermodifizierte Dimethylpolysiloxane und Alkylbenzolsulfonate.

Weitere Hilfsstoffe in den erfindungsgemäßen Präparaten können übliche Stoffe zur Veränderung der rheologischen Eigenschaften des Präparats, oberflächenaktive Mittel, die Haftung verstärkende Hilfsstoffe sowie Trocknungsbeschleuniger im Falle der Verwendung eines UV-härtbaren Harzes. Die zusätzliche Verwendung einer wäßrigen Polysulfidlösung hat sich in manchen Fällen als zweckmäßig erwiesen.

Wäßrige und wäßrig-organische Glanzgoldpräparate, enthaltend mindestens eine erfindungsgemäße schwefelorganische Goldverbindung, enthalten letztere in einer Menge von 2 bis 25 Gew.-%, vorzugsweise 5 bis 15 Gew.-%, jeweils berechnet als Gold. Der Bindemittelanteil in den Präparaten liegt im allgemeinen zwischen 5 und 45 Gew.-%, wobei das Gewichtsverhältnis von Harz zu Edelmetall vorzugsweise zwischen 0,5 und 1 bis 1,5 zu 1 liegt. Der Wassergehalt liegt im allgemeinen zwischen 10 und 90 Gew.-%, vorzugsweise zwischen 30 und 70 Gew.-%, und der Gehalt an organischen Lösungsmitteln zwischen 0 und 40 Gew.-%, jeweils bezogen auf das Präparat. Die Einsatzmenge der Flußmittel bewegt sich üblicherweise im Bereich zwischen 0,01 und 2 Gew.-%, bezogen auf das Präparat. Im Falle von sogenannten Poliergoldpräparaten enthalten diese zusätzlich Goldpulver und/oder feinteilige anorganische unlösliche Goldverbindungen. Je nach gewünschtem Effekt können Poliergoldpräparate zusätzlich noch Glasfritten und/oder Organosiliciumverbindungen enthalten.

Die mindestens eine schwefelorganische Goldverbindung enthaltenden Präparate können unmittelbar mittels üblicher Dekorationsverfahren, wie Sprühen, Pinseln und bekannter Druckverfahren, insbesondere Siebdruckverfahren, auf die zu dekorierende Oberfläche aufgebracht und nach dem Abdunsten der Lösungsmittelbestandteile das Dekor bei einer Temperatur zwischen im allgemeinen 400 und 900 °C eingebrannt werden.

Eine in der keramischen Industrie übliche Dekorationstechnik besteht auch in der Verwendung von Abziehbildern. Demgemäß lassen sich die erfindungsgemäßen schwefelorganischen Goldverbindungen auch in der Dekorschicht derartiger Abziehbilder einsetzen. Die Herstellung des Abziehbilds erfolgt in für den Fachmann bekannter Weise: Auf eine auf einen Träger aufgebrachte wasserlösliche Trennschicht oder eine Thermotrennschicht wird eine Dekorschicht aufgebracht, deren Zusammensetzung im wesentlichen derjenigen entspricht, wie sie in den zuvor beschriebenen Präparaten eingesetzt wird. Nach Abdunsten des Lösungsmittels, bei den bevorzugten Präparaten also im wesentlichen Wasser, und, sofern erforderlich, Vernetzen des Bindemittels wird die Dekorschicht üblicherweise überfilmt.

Durch die Erfindung wurde eine völlig neue Stoffklasse schwefelorganischer Goldverbindungen zur Verfügung gestellt, welche nicht nur in konventionellen organischen oder organisch-wäßrigen Präparaten zur Herstellung von Golddekoren auf einbrennfähigen Unterlagen geeignet sind, sondern auch in im wesentlichen rein wäßrigen Präparaten zur Anwendung gelangen können. Unter Verwendung der neuen schwefelorganischen Goldverbindungen in rein wäßrigen Präparaten lassen sich hochglänzende und gut haftende Golddekore herstellen. Durch derartige wäßrige Präparate werden Nachteile, wie sie bei der Verwendung organischer Lösungsmittel enthaltender Präparate zwangsweise auftreten, überwunden.

### Beispiel 1

### Herstellung von Monogold(I)-dimercaptobernsteinsäure (Au(I)-dmbs) (Ausgangsverbindung zur Herstellung erfindungsgemäßer Goldverbindungen)

0,035 mol = 18,49 g HAuCl₄ (37,29 % Au) wird unter Rühren zu einer Suspension von 0,07 mol Methionin in 70 ml H₂O getropft. Durch externe Kühlung wird die Temperatur auf 0 bis 5 °C gehalten. Nach Beendigung der Reaktion wird der Gold(I)-Komplex zu einer Suspension von 0,035 Mol = 6,38 g meso-2,3-Dimercaptobernsteinsäure in 150 ml Dichlormethan innerhalb einer Stunde getropft. Der Niederschlag wird abgesaugt, mehrmals mit Wasser gewaschen und im Exikkator unter Vakuum über Blaugel getrocknet. Die Ausbeute an Monogolddimercaptobernsteinsäure beträgt 96,5 %, bezogen auf das eingesetzte Gold.

| Analyse: | Au | C | H | S |
|---|---|---|---|---|
| berechnet: | 52,09 % | 12,70 % | 1,33 % | 16,96 % |
| gefunden: | 50,81 % | 12,05 % | 1,52 % | 15,62 % |

¹³C-NMR in D₂O + Trimethylamin δ/ppm 52,58 (CH), 60,95 (CH), 180,64 (CO₂⁻) und 182,51 (CO₂⁻)

Figur 2 zeigt das Voltammogramm von Au-dmbs (in 0,05 nKOH) aus der polarographischen Bestimmung an einer Hg-Tropfelektrode gegen Ag/AgCl als Bezugselektrode. Die Abszisse reicht von -100 mV bis -1800 mV, die Ordinate zeigt den Strom in µA (mikro A). Aus dem Kurvenverlauf folgt, daß bei -700 mV Au⁺¹ zu Au⁰ reduziert wird.

Figur 4 zeigt das IR-Spektrum der Monogold(I)-dimercaptobernsteinsäure (in KBr-Tablette).

Aus dem Röntgenphotoelektronenspektrum XPS/ESCA (Gerät: Leybold MAX 100) von Au(I)-dmbs folgt als Maximum des Au4f_{7/2}-Peaks : 84,46 eV

### Beispiel 2

6 g Au(I)-dmbs aus Beispiel 1 werden in 20 ml Wasser und ca. 3 g Triethylammin bei 60 °C gelöst. Anschließend stellt man mit konz. HCl die Lösung auf pH 2 ein. Der sich bildende Niederschlag wird abgesaugt und mit Wasser gewaschen. Der Filterrückstand wird getrocknet.
Ausbeute: 2,6 g schwarze Substanz

| | | |
|---|---|---|
| Anlayse: | AU | 68,34 % |
| | C | 9,07 % |
| | H | 1,42 % |
| | S | 10,74 % |
| | N | 0,55 % |

Atomverhältnis Au/S = 1,04 : 1
Zum Vergleich: Au(I)-dmbs enthält 52,09 % Au und ein Au/S-Atomverhältnis von 0,5.

### Beispiel 3

2,1 g der in Beispiel 2 hergestellten Verbindung werden in 15 ml Wasser und Triethylammin bei 60 °C gelöst. Anschließend stellt man mit konz. HCl die Lösung auf pH 2 ein. Der sich bildende Niederschlag wird abgesaugt und mit Wasser gewaschen. Der Filterrückstand wird getrocknet.
Ausbeute: 0,88 g schwarze Substanz

| | | |
|---|---|---|
| Analyse: | Au | 73,99 % |
| | C | 7,12 % |
| | H | 1,20 % |
| | S | 10,04 % |
| | N | 0,57 % |

Atomverhältnis Au/S = 1,20 : 1

Figur 1 zeigt das Voltammogramm des in Beispiel 3 erhaltenen Produkts (in 0,05 nNaOH) aus der polarographischen Bestimmung an einer Hg-Tropfelektrode gegen Ag/AgCl als Bezugselektrode. Abszisse von -100 mV bis -1800 mV; Ordinate in nA (nano-A). Aus dem Kurvenverlauf folgt, daß bis -1800 mV, von minimalen Verunreinigungen zwischen -400 und -800 mV abgesehen, bis -1800 mV keine Reduktion erfolgt.

Figur 3 zeigt das IR-Spektrum des Produkts aus Beispiel 3, das vollständig von demjenigen des Ausgangsprodukts (Fig. 4) abweicht.

Aus dem Röntgenphotoelektronenspektrum XPS/ESCA (Gerät: Leybold MAX 100) folgt als Maximum des Au4f_{7/2}-Peaks 84,2 eV (zum Vergleich: Au(I)-dmbs 84,46 eV, Au metallisch 84,0 eV). Aus der quantitativen Auswertung des XPS-Spektrums, das nur die obersten Atomlagen des Materials erfaßt,
resultiert folgende Analyse:

| | |
|---|---|
| O | 12,79 Atom-% |
| C | 49,68 " |
| S | 13,82 " |
| Au | 23,72 " |

### Beispiel 4

50 g Au(I)-dmbs werden in 400 ml Wasser und einer entsprechenden Menge an Triethylammin bei RT (= Raumtemperatur) gelöst, so daß sich in der Lösung ein pH-Wert von größer 10 einstellt. Die Lösung wird dann mit konz. HCl auf pH 2 eingestellt. Den sich bildenden Niederschlag filtriert man und löst diesen in Wasser und Triethylammin bei RT, wobei pH-Wert von 10 eingestellt wird. Anschließend wurde mit konz. HCl auf pH 2 angesäuert. Der Niederschlag wird filtriert und getrocknet.
Ausbeute: 21,42 schwarze Substanz

| | | |
|---|---|---|
| Analyse: | Au | 70,40 % |
| | C | 8,25 % |
| | H | 1,30 % |
| | S | 10,58 % |
| | N | 0,74 % |

Atomverhältnis Au/S = 1,08 : 1

### Beispiel 5

7 g der Verbindung aus Beispiel 4 werden in 25 ml Wasser und einer entsprechenden Menge an Triethylammin bei RT gelöst. Anschließend wurde mit konz. HCl auf pH 1-2 angesäuert. Der Niederschlag wird filtriert und getrocknet.
Ausbeute: 6,15 g schwarze Substanz

| | | |
|---|---|---|
| Analyse: | Au | 77,28 % |
| | C | 6,25 % |
| | H | 0,95 % |
| | S | 10,25 |
| | N | 0,69 |

Atomverhältnis Au/S = 1,23 : 1

### Beispiel 6

10 g Au(I)-dmbs (26 mmol) werden in 200 ml Wasser und 8 g Triethylammin (79 mmol) bei RT gelöst. Die Lösung wird dann mit 6,6 ml konz. HCl ca. (79 mmol) versetzt. Der gebildete Niederschlag wird filtriert und getrocknet.
Ausbeute: 7,83 g braune Substanz

| | | |
|---|---|---|
| Analyse: | Au | 60,90 % |
| | C | 11,45 % |
| | H | 1,62 % |
| | S | 12,57 % |
| | N | 0,50 % |

Atomverhältnis Au/S = 0,78 : 1

### Beispiel 7

5 g des Produktes von Beispiel 6 (enthält 15 mmol Au) werden in 100 ml Wasser und 4,5 g Triethylammin (45 mmol) bei 60 °C gelöst. Die Lösung wird dann mit 3,8 ml konz. HCl ca. (45 mmol) versetzt. Der gebildete Niederschlag wird filtriert und getrocknet.
Ausbeute: 3,43 g schwarze Substanz

| | | |
|---|---|---|
| Anlayse: | Au | 74,28 % |

Der Beginn der thermodynamischen Zersetzung von der schwarzen Substanz liegt bei 173 °C und damit deutlich unterhalb des Zersetzungsbeginns von Au(I)dmbs (194 °C).

### Beispiel 8: Glanzgoldpräparat

Folgende Bestandteile wurden gemischt:

| | Teile |
|---|---|
| Polyvinylpyrrolidon (PVP K25 von Fluka) | 5,2 |
| Goldverbindung aus Beispiel 4 | 13,4 |
| 1,2-Propandiol | 6,1 |
| Wasser | 41,9 |
| Ethyllactat | 25,1 |
| n-Butanol | 0,9 |
| Rhodium-Komplex | 0,12 |
| Chromsulfat | 0,33 |
| Ammoniumwismutcitrat | 0,44 |
| Triethylamin | 4,4 |
| Ammoniumpolysulfid*) | 2,1 |

| | |
|---|---|
| *) 15 Gew.-%ige wäßrige Lösung; gilt auch für Beispiele 9 und 10. | |

Das Präparat wird per Siebdruck auf Porzellan aufgetragen und 10 min bei 820 °C erhitzt, wobei die Aufheizzeit 1 h beträgt. Man erhält einen glänzenden, gut haftenden Film.

### Beispiel 9: Glanzgoldprodukt

Folgende Bestandteile wurden gemischt:

| | Teile |
|---|---|
| PVP K25 | 8,3 |
| Goldverbindung aus Beispiel 5 | 12,6 |
| 1,2-Propandiol | 8,3 |
| Wasser | 43,4 |
| Ethyllactat | 16,6 |
| Rhodium-Komplex | 0,15 |
| Chromsulfat | 0,04 |
| Ammoniumwismutcitrat | 0,49 |
| Triethylamin | 5,5 |
| Ammoniumpolysulfid*) | 2,0 |
| Silbermercaptopropionylglycin | 2,6 |

| | |
|---|---|
| *) 15 Gew.-%ige wäßrige Lösung; gilt auch für Beispiele 9 und 10. | |

Das Präparat wird per Siebdruck auf Porzellan aufgetragen und 2 min bei 880 °C erhitzt, wobei die Aufheizzeit 30 min beträgt. Man erhält einen glänzenden, gut haftenden Film.

### Beispiel 10: Glanzgoldpräparat

Folgende Bestandteile wurden gemischt:

| | Teile |
|---|---|
| PVP K25 | 5,2 |
| Goldverbindung aus Beispiel 4 | 13,3 |
| Wasser | 73,3 |
| Rhodium-Komplex | 0,12 |
| Chromsulfat | 0,33 |
| Ammoniumwismutcitrat | 0,43 |
| Triethylamin | 5,2 |
| Ammoniumpolysulfid*) | 2,1 |

| | |
|---|---|
| *) 15 Gew.-%ige wäßrige Lösung; gilt auch für Beispiele 9 und 10. | |

Das Präparat wird auf das Porzellan gepinselt oder mittels Siebdruck aufgetragen und 10 min bei 820 °C erhitzt, wobei die Aufheizzeit 1 h beträgt. Man erhält einen glänzenden, gut haftenden Film.

### Beispiel 11

Herstellung einer erfindungsgemäßen Goldverbindung, ausgehend von Monogold(I)-dimercaptobernsteinsäure (Au(I)dmbs).

Au(I)dmbs mit einem Au-Gehalt von 52,09 % wird unter Zugabe von Tetraethylammoniumhydroxid in Wasser gelöst. Nach Zugabe von konz. HCl fällt eine Goldverbindung mit einem Au-Gehalt 62,89 % aus (11/1). Durch analoges Auflösen des Produktes 11/1 und Ausfällen steigt der Au-Gehalt auf 68,63 % (11/2) und durch Auflösen von 11/2 und Ausfällen auf 74,68 % (11/3) an. Das Gold-Schwefel-Verhältnis steigt von 0,54 : 1 auf 0,89 : 1 (=11/1), dann auf 1,11 : 1 (=11/2) und schließlich auf 1,25 : 1 (=11/3) an.

Die Goldverbindung 11/3 wurde im Glanzgoldpräparat des Beispiels 8 anstelle der dort eingesetzten Au-Verbindung getestet. Erhalten wurde ein schöner, festhaftender, hochglänzender Goldfilm.

### Beispiel 12

Au(I)dmbs wird zunächst unter Zugabe von 2 mMol Triethylamin pro mMol Au(I)dmbs in Wasser gelöst. Anschließend werden 3 mMol Tetramethylammoniumchlorid zugegeben. Nach Zugabe von konz. HCl fällt eine erfindungsgemäße Goldverbindung mit einem Au-Gehalt von 71,31 % aus; das Au/S-Verhältnis stieg von 0,54 auf 1,08 an.

Die Au-verbindung wurde als Komponente in einem Glanzgoldpräparat analog Beispiel 9 getestet. Man erhält einen schönen, festhaftenden, hochglänzenden Goldfilm.

## Patentansprüche

1. Schwefelorganische Goldverbindung mit einem Goldgehalt im Bereich von 60 bis 90 Gew.-% und einem Atomverhältnis von Gold zu Schwefel von größer 0,71 bis 4, erhältlich durch ein Verfahren mit den Stufen
(i) Auflösen einer Monogold(I)-dimercaptocarbonsäureverbindung der allgemeinen Formel (A) 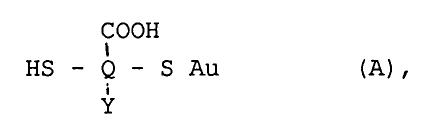 worin Q einen aliphatischen C₂- bis C₄-Alkantetraylrest oder einen 5- oder 6-gliedrigen, gegebenenfalls ein Sauerstoff- oder Imin-Ringglied enthaltenden cycloaliphatischen Tetraylrest und Y Wasserstoff oder COOH bedeuten und die S-Atome an benachbarten C-Atomen stehen, oder ein Salz der Verbindung (A) in Wasser in Gegenwart einer Base,
(ii) Zugabe einer Mineralsäure zur Lösung der Stufe (i) bis zu einem pH-Wert von gleich oder unter 2 und
(iii) Isolierung des in Stufe (ii) ausgefallenen Produkts.

2. Schwefelorganische Goldverbindung nach Anspruch 1,
dadurch gekennzeichnet,
daß sie in Wasser, gegebenenfalls nach Zugabe einer Base, löslich ist.

3. Schwefelorganische Goldverbindung nach Anspruch 1 oder 2,
dadurch gekennzeichnet,
daß sie 68 bis 85 Gew.-% Gold enthält und ein Atomverhältnis von Gold zu Schwefel von größer 1 bis 3 aufweist.

4. Verfahren zur Herstellung schwefelorganischer Goldverbindungen gemäß einem der Ansprüche 1 bis 3,
dadurch gekennzeichnet,
daß es die Stufen
(i) Auflösen einer Monogold(I)-dimercaptocarbonsäureverbindung der allgemeinen Formel (A) 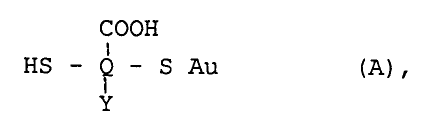 worin Q einen aliphatischen C₂- bis C₄-Alkantetraylrest oder einen 5- oder 6-gliedrigen, gegebenenfalls ein Sauerstoff- oder Imin-Ringglied enthaltenden cycloaliphatischen Tetraylrest und Y Wasserstoff oder COOH bedeuten und die S-Atome an benachbarten C-Atomen stehen, oder ein Salz der Verbindung (A) in Wasser in Gegenwart einer Base,
(ii) Zugabe einer Mineralsäure zur Lösung der Stufe (i) bis zu einem pH-Wert von gleich oder unter 2 und
(iii) Isolierung des in Stufe (ii) ausgefallenen Produkts,
umfaßt.

5. Verfahren nach Anspruch 4,
dadurch gekennzeichnet,
daß man die Stufen (i) bis (iii) ein oder mehrfach wiederholt, wobei als Ausgangsprodukt für die Stufe (i) der zweiten und folgenden Sequenzen jeweils das in der Stufe (iii) der vorhergehenden Sequenz isolierte Produkt eingesetzt wird.

6. Verfahren nach einem der Ansprüche 4 bis 5,
dadurch gekennzeichnet,
daß man als Base ein Amin und/oder ein Tetraalkylammoniumhydroxid verwendet.

7. Verfahren nach einem der Ansprüche 4 bis 6,
dadurch gekennzeichnet,
daß man der Stufe (ii) eine wäßrige Lösung der Goldverbindung der Formel A, welche durch zugesetztes Tetraalkylammoniumhydroxid oder ein Tetraalkylammoniumsalz Tetraalkylammoniumionen enthält, zuführt.

8. Verfahren nach einem der Ansprüche 4 bis 7,
dadurch gekennzeichnet,
daß man in der Stufe (i) Monogold(I)-dimercaptobernsteinsäure in Wasser auflöst, indem man ein Tri(C₁- bis C₃)alkylamin bis zu einem pH-Wert von mindestens 8, vorzugsweise 9 bis 11, zugibt.

9. Verwendung der schwefelorganischen Goldverbindungen gemäß einem der Ansprüche 1 bis 3 zur Herstellung eines Mittels zur Erzeugung eines Golddekors auf einbrennfähigen Unterlagen.

10. Goldpräparat zur Herstellung von Glanzgold- und Poliergolddekoren, enthaltend eine schwefelorganische Goldverbindung gemäß einem der Ansprüche 1 bis 3, in gelöstem Zustand, ein im wesentlichen wäßriges, organisches oder wäßrig-organisches Lösungsmittelsystem und ein polymeres Bindemittelsystem.

## Claims

1. Organosulfur gold compound having a gold content in the range from 60 to 90 wt.% and an atomic ratio of gold to sulfur of greater than 0.71-4, obtainable by a process comprising the stages
(i) dissolution of a monogold(I) dimercaptocarboxylic acid compound of the general formula (A) 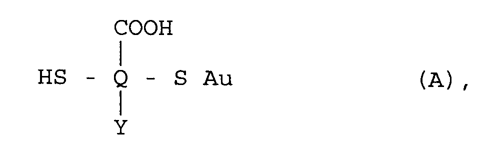 in which Q means an aliphatic C₂ to C₄ alkanetetrayl residue or a 5- or 6-membered cycloaliphatic tetrayl residue optionally containing an oxygen or imine ring member and Y means hydrogen or COOH and the S atoms are located on adjacent C atoms, or a salt of a compound (A) in water in the presence of a base,
(ii) addition of a mineral acid to the solution of stage (i) down to a pH value of less than or equal to 2 and
(iii) isolation of the product precipitated in stage (ii).

2. Organosulfur gold compound according to claim 1,
characterised in that
it is soluble in water, optionally after addition of a base.

3. Organosulfur gold compound according to claim 1 or 2,
characterised in that
it contains 68 to 85 wt.% of gold and has an atomic ratio of gold to sulfur of greater than 1-3.

4. Process for the production of organosulfur gold compounds according to one of claims 1 to 3,
characterised in that
it comprises the stages
(i) dissolution of a monogold(I) dimercaptocarboxylic acid compound of the general formula (A) 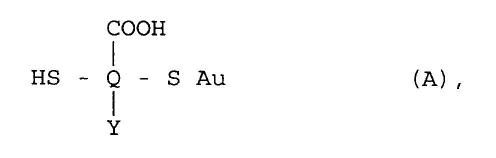 in which Q means an aliphatic C₂ to C₄ alkanetetrayl residue or a 5- or 6-membered cycloaliphatic tetrayl residue optionally containing an oxygen or imine ring member and Y means hydrogen or COOH and the S atoms are located on adjacent C atoms, or a salt of a compound (A) in water in the presence of a base,
(ii) addition of a mineral acid to the solution of stage (i) down to a pH value of less than or equal to 2 and
(iii) isolation of the product precipitated in stage (ii).

5. Process according to claim 4,
characterised in that
stages (i) to (iii) are repeated once or more, wherein the starting product for stage (i) in the second and subsequent sequences is in each case the product isolated in stage (iii) of the preceding sequence.

6. Process according to one of claims 4 to 5,
characterised in that
the base used is an amine and/or a tetraalkylammonium hydroxide.

7. Process according to one of claims 4 to 6,
characterised in that
an aqueous solution of the gold compound of the formula A which contains tetraalkylammonium ions from the addition of tetraalkylammonium hydroxide or a tetraalkylammonium salt is introduced into stage (ii).

8. Process according to one of claims 4 to 7,
characterised in that
monogold(I) dimercaptosuccinic acid is dissolved in water in stage (i) by adding a tri(C₁ to C₃)alkylamine up to a pH value of at least 8, preferably of 9 to 11.

9. Use of the organosulfur gold compounds according to one of claims 1 to 3 for the production of an agent for the production of gold decoration on stovable substrates.

10. Gold preparation for the production of bright gold and burnished gold decorations containing an organosulfur gold compound according to one of claims 1 to 3, in the dissolved state, a substantially aqueous, organic or aqueous/organic solvent system and a polymeric binder system.

## Revendications

1. Composé d'or organique, soufré, ayant une teneur en or dans la zone de 60 à 90 % en poids et un rapport atomique de l'or au soufre supérieur à 0,71 à 4, accessible par un procédé comportant les étapes :
(i) mise en solution d'un composé acide monoauro (I) dimercapto carboxylique de formule générale (A) : 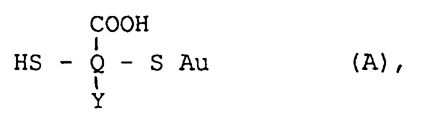 dans laquelle Q signifie un radical alkanetétrayl en C₂ à C₄ aliphatique ou un radical tétrayl cycloaliphatique contenant un élément de cycle à 5 ou 6 membres, éventuellement un élément de cycle oxygéné ou iminé, et Y signifie de l'hydrogène ou COOH, et les atomes de S se situent sur les atomes de C voisins, ou un sel du composé A, dans l'eau en présence d'une base, de préférence une amine,
(ii) addition d'un acide minéral à la solution de l'étape (i) jusqu'à une valeur de pH égale ou inférieur à 2,
(iii) isolement du produit précipité au stade (ii).

2. Composé d'or organique soufré selon la revendication 1,
caractérisé en ce qu'
il est soluble dans l'eau, éventuellement après addition d'une base.

3. Composé d'or organique soufré selon la revendication 1 ou la revendication 2,
caractérisé en ce qu'
il renferme de 68 à 85 % en poids d'or et qu'il possède un rapport atomique de l'or au soufre allant de plus de 1 à 3.

4. Procédé de préparation de composés d'or organiques soufrés conformément à l'une des revendications 1 à 3,
caractérisé en ce qu'
il comprend les étapes de :
(i) mise en solution d'un composé acide monoauro (I) dimercapto carboxylique de formule générale (A) : 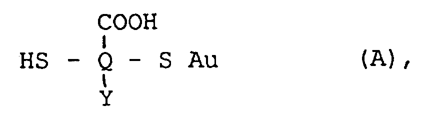 dans laquelle Q signifie un radical alkanetétrayl en C₂ à C₄ aliphatique ou un radical tétrayl cycloaliphatique contenant un élément de cycle à 5 ou 6 membres, éventuellement un élément de cycle oxygéné ou iminé, et Y signifie de l'hydrogène ou COOH, et les atomes de S se situent sur les atomes de C voisins,
ou un sel du composé A,
dans l'eau en présence d'une base,
(ii) addition d'un acide minéral à la solution de l'étape (i) jusqu'à une valeur de pH égale ou inférieure à 2,
(iii) isolement du produit précipité au stade (ii).

5. Procédé selon la revendication 4,
caractérisé en ce qu'
on répète les étapes (i) à (iii) une ou plusieurs fois, dans lesquelles on met en oeuvre comme produit de départ pour l'étape (i) des deuxièmes et suivantes séquences, à chaque fois le produit isolé dans la séquence (iii) de la séquence précédente.

6. Procédé selon l'une des revendications 4 à 5,
caractérisé en ce qu'
on utilise comme base une amine et/ou un hydroxyde de tétraalkylammonium.

7. Procédé selon l'une des revendications 4 à 6,
caractérisé en ce qu'
on amène au stade (ii) une solution aqueuse du composé d'or de formule A, qui contient des ions tétraalkylammonium ou à travers un hydroxyde tétraalkyl ammoniun un sel de tétraalkylammonium.

8. Procédé selon l'une des revendications 4 à 7,
caractérisé en ce qu'
on met en solution à l'étape (i) l'acide monoauro(I)dimercaptosuccinique dans l'eau, procédé dans lequel on ajoute une tri(alkyl en C₁ à C₃) amine jusqu'à une valeur de pH d'au moins 8, de préférence de 9 à 11.

9. Utilisation des composés d'or organique soufrés conformément à l'une des revendications 1 à 3, en vue de la préparation d'un produit pour l'obtention d'un décor doré sur des substrats aptes à la cuisson.

10. Préparation d'or pour la production de décors d'or imitation et d'or de polissage contenant un composé d'or organique soufré conformément à l'une des revendications 1 à 3, à l'état dissout, un système de solvants essentiellement aqueux, organique ou aquo-organique et un système d'agents liants polymères.
